# EUROPEAN PATENT SPECIFICATION

(11) **EP 2 272 375 B1**
(45) Date of publication and mention of the grant of the patent: **23.10.2013**
(21) Application number: 10175609.6
(22) Date of filing: 26.07.2006
(51) Int. Cl.: A23C 19/032, C12R 1/80

(54) **"Gluten-free" blue-veined dairy products intended for persons affected by celiac disease**
"Gluten-freie" Blauschimmel-enthaltende Milchprodukte für unter Zoeliakie leidende Personen
Produits laitiers à pâte persillée sans gluten pour personnes atteintes de la maladie coeliaque

(30) Priority: 11.10.2005 IT MI20051908; 23.06.2006 IT MI20061211
(43) Date of publication of application: 12.01.2011
(62) Divisional of application: 06780601.8
(73) Proprietor: Mofin S.R.L., 28100 Novara (IT)
(72) Inventor: Mogna, Giovanni, 28100 Novara (IT); Strozzi, Gian Paolo, 28100 Novara (IT); Bruno, Federico, 28060 Granozzo con Monticello Frazione Case Sparse (IT)
(74) Representative: HOFFMANN EITLE

(56) References cited:
- FR-A1- 2 447 682
- US-A1- 2005 249 719
- IAMETTI F ET AL: "Determination of the gluten residue content of fungal cultures used in the production of Gorgonzola cheese" IL LATTE, TECNICHE NUOVE, MILAN, IT, vol. 25, no. 10, 1 January 2000 (2000-01-01), pages 52-56, XP008071932 ISSN: 0392-6060
- BLASZYK M ET AL: "Reduced water activity during sporogenesis in selected penicillia: Impact on spore quality" FOOD RESEARCH INTERNATIONAL, vol. 31, no. 6-7, 1998, pages 503-509, XP002409104 ISSN: 0963-9969
- DEMYTTENAERE J C R ET AL: "De novo production of (+)-aristolochene by sporulated surface cultures of Penicillium roqueforti" PHYTOCHEMISTRY, PERGAMON PRESS, GB, vol. 59, no. 6, March 2002 (2002-03), pages 597-602, XP004339152 ISSN: 0031-9422
- STRATEGIES FOR SPORE PRODUCTION BY PENICILLIUM-ROQUEFORTII USING SOLID STATE FERMENTATION TECHNIQUES: "STRATEGIES FOR SPORE PRODUCTION BY PENICILLIUM-ROQUEFORTII USING SOLID STATE FERMENTATION TECHNIQUES" PROCESS BIOCHEMISTRY, ELSEVIER, NL, vol. 24, no. 3, 1989, pages 97-103, XP008071904 ISSN: 1359-5113
- ALAEDINI ARMIN ET AL: "Narrative review: Celiac disease: Understanding a complex autoimmune disorder" ANNALS OF INTERNAL MEDICINE, vol. 142, no. 4, 15 February 2005 (2005-02-15), pages 289-298, XP002414042 ISSN: 0003-4819

## Description

The present invention relates to "gluten-free" spore or mildew cultures of the genus *Penicillium*, in particular of the species *Penicillium roqueforti*, for the blue-veining of cheeses intended for persons affected by celiac disease.

The aim of the present invention is a method for the preparation of said gluten-free cultures which foresees the use of a gluten-free anallergic culture substrate, possibly in association with a subsequent enzymatic treatment suitable for the complete elimination of gluten traces, if any, deriving from accidental and unintentional contaminations (cross-contaminations) .

The celiac disease is an enterophatic, chronic, immune-mediated inflammatory disease, risen from the ingestion of gluten, a "storage" protein naturally contained in some cereals. This food intolerance affects genetically predisposed persons, of any age, having an immune system which responds in an abnormal way to the ingestion of proteinic fractions typical of the wheat gluten, spelt (a kind of wheat), kamut and spel (a kind of wheat), barley, rye, triticale (a cross between wheat and rye) and their derivatives. Some individuals also present intolerance to the oat proteins. Technically, the term "gluten" applies for the combination of the simple prolaminic (rich in proline), called "gliadins", and glutelinic (rich in glutamine), called "glutenins" proteins of the cereals above mentioned. In the context of the celiac disease, the term "gluten" is often used with reference to all kinds of proteins contained in the cereals, even if between the different proteinic fractions which form the gluten, the gliadin seems to be the most detrimental. The celiac disease can occur in multiple clinical forms, not always in a manifest way; in fact, there are latent or potential, clinically silent and without evident symptoms, mono-symptomatic forms, or with a full, intestinal and extra-intestinal clinical symptom.

The chronic inflammation leads to the increase of the intestinal intraepithelial lymphocytes, to the crypts hypertrophy and the progressive flattening and disappearance of the intestinal villa of the small intestine, in particular the duodenal mucosa, with a consequent loss of the ability of absorbing the nutritive substances and occurrence of symptoms such as chronic diarrhea, abdominal distension, inappetence, weight decrease, anaemia and vitamin deficiencies. In the most serious cases, countermarked with a late diagnosis, osteoporosis, infertility, repeated abortions, low height in the young people, diabetes mellitus, autoimmune thyroiditis, alopecia, epilepsy with cerebral calcifications and the fearful intestinal lymphoma can occur. In the celiac persons, the intake of gluten, also in small quantities, is capable of causing the abnormal response of the immune system: the transglutaminase, an enzyme existing in the intestinal mucous tissue, binds itself to the gliadin and through deamidation transforms it in a molecule capable of activating the T cells (cells of the immune system capable, of mediating all the immune responses towards the proteinic antigens), with a consequent production of anti-transglutaminase IgG and IgA and anti-endomise IgA immunoglobulins. In a first stage, an increase of the intestinal intraepithelial activated T cells occurs, while with the progress of the disease the increase relates both to the lymphocytes and the infiltrated plasma cells of the own lamina, with a production of metalloproteinases responsible for the shortening of the villi and therefore the damage to the intestinal mucosa.

The possibility of preventing the development or treating the celiac disease does not exist so far, therefore a gluten-free diet, strictly carried out throughout the life is the only therapy capable of ensuring to the celiac persons a perfect health. The celiac persons must also eliminate the smallest traces of flour of the dangerous cereals, because the intake of gluten, also in minimal quantities, can unbridle the autoimmune response. As the ratio of the quantity of ingested gluten to the toxic effect induced at the intestinal level has still not been defined, the term "traces", from the quantitative point of view, has a fundamental practical importance in the treatment of the celiac disease and implications on the food legislation plan, because said "traces" are related to the maximum limit of "acceptable" gluten (threshold) in the products suitable for the diet of the celiac person. All the skilled persons agree that a dose of 100 mg per day of gliadin, equal to 200 mg of gluten, i.e. about 3 g of bread, is sufficient to cause, in most of the celiac persons, the increase of the intestinal intraepithelial lymphocytes, a premature sign of persistent intestinal inflammation.

With reference to the minimum threshold, the few scientific works carried out so far would seem to show that the ingestion up to 10 milligrams per day of gliadin (equal to 20 parts per million of gluten) is not able to sensitively damage the intestinal mucosa, but can determine, in a minority of the cases, the occurrence of gastroenteric symptoms.

At the international legislative level, the old standard "Codex Standard for Gluten-Free Foods", moreover still in force, shows, as the maximum gluten content in the diet-therapeutic products, 0.05 of nitrogen per 100 g of dry product (with reference to the wheat starch), which corresponds to a gluten fraction equal to about 500 ppm. However, a right and proper review of the aforesaid guide line is taking place, which seems to foresee that the gluten-free foods resulting from naturally gluten-free ingredients have not to contain more than 20 ppm of gluten, while the gluten-free foods deriving from cereals with gluten, can have a maximum limit of 200 ppm of gluten.

In France, Great Britain and the Netherlands, waiting for the review of the aforesaid guide line, they consider, as the maximum limit for the gluten-free products, 200 parts per million. In Italy, a law (Legislative Decree n. 109/1992) will come into force, which establishes that, if in the composition of the food product or in that of one or more ingredients (flavourings, additives or co-adjuvants) which form the same, are present cereals containing gluten or substances deriving therefrom and/or if from the productive process a quantity of gluten, analytically determined as higher than 20 parts per million, can derive in the end product such product will have to show in the label, at the foot of the ingredients list and in a well visible way, the words "gluten-containing product".

Therefore, in order to ensure the celiac persons a precise information on the absence of gluten in the foods, it will be mandatory, also in the cheeses, to show in the label the presence of possible gluten traces .

For the dairy industry specialized in the production of blue-veined cheeses, this new regulating aspect involves a serious commercial damage deriving from the decrease of the potential circle of consuming buyers. The complete exclusion of the gluten from the diet, however, is not easy to carry out, considering that cross-contamination phenomena of cereals and derivatives (starches, flours, starch flour, etc.) naturally gluten-free, already at the milling industry level, can occur. Such products are then used by the food industry in the preparation of complex foods based on multiple technological ingredients, additives and co-adjuvants of different origin and nature. A recent research has shown that up to 6% of the "theoretically" gluten-free products, based on the reported ingredients, actually contain over 30 mg of gliadin per 100 g of end product, equal to 600 ppm of gluten. For the purpose of excluding a possible gluten-contamination, it is therefore necessary to consider, for each commercialized food product, not only all the ingredients used and the processing, but also all the productive chain of each single ingredient.

Among the "foods at risk", as also signalled by the Italian Association of Celiac persons, the "Blue- veined cheeses and with a mouldy rind" are further included, since from experimental investigations, carried out also in Italy, it results that some "blue-veined cheeses and with a mouldy rind" can contain gluten deriving from the growth culture media of schizomycetes (mildews and yeasts) used as starter cultures in order to obtain the typical blue-veining of the blue cheese (Penicillium *roqueforti*) , the white felt coating of cheeses such as Brie, Camembert and Carre de l'Est, Tomme blanche (*Penicillium candidum*) and/or the refining of many other kinds of cheese (Kluyveromices, Debaromyces genera) .

According to the rule which will come into force, born in order to satisfy the need of the celiac persons of a precise information about the absence of gluten in the foods, if such cheeses contain gluten traces, or however are manufactured with technological gluten- containing ingredients or co-adjuvants, it will be obligatory to show it in the label.

The document IAMMETTI F. ET AL: "Determinazione del contenuto glutinico residuo in colture fungine utilizzate per la produzione di Gorgonzola", IL LATTE, TECNICHE NUOVE, MILANO, IT, vol. 25, no. 10, 2000-01-01, pages 52-56, relates to the determination of the amount of gluten in two *Penicillium* coltures by using an ELISA test.

The French patent application FR 2447682 A1 relates to the germination of a *Penicillium* roqueforti culture on a medium containing whey powder (*poudre* de *lactoserum*).

The document BLASZYK M. ET AL.: "Reduced water activity during sporogenesis in selected penicillia: Impact on spore quality", FOOD RESEARCH INTERNATIONAL, vol. 31, no. 6-7, 1998, pages 503-509, relates to the use of PDA (potato, dextrose agar) medium for the *Penicillum roqueforti* spore production.

The document DEMYTTENAERE J. C. R. ET AL.: "De novo production of (+)-aristolochene by sporulated surface cultures of Penicillium roqueforti", PHYTOCHEMISTRY, PERGAMON PRESS, GB, vol. 59, no. 6, March 2002, pages 597-602, relates to the use of PDA (potato, dextrose agar) medium for the *Penicillum roqueforti* spore production.

Therefore, there remains a strong need of being able to provide "gluten-free" blue-veined dairy products. Also in the case in which the manufacturing process of the Penicillium cultures does not foresee the use of any gluten-containing ingredient or substance, it is however possible that, because of unintentional and accidental cross-contaminations, some components used in the productive process brings gluten traces. Therefore, all the sector operators agree that, so far, there remains a very strong need of providing a method for the preparation of culture media suitable for the growth of the schizomycetes (mildews or yeasts), used as starter cultures in order to obtain the blue-veining of the cheeses, capable of using a substrate not based on bread and, simultaneously, capable of reducing the cross-, unintentional and accidental contaminations, should they occur. In particular, there remains the need of providing a method for the preparation of growth culture media of the schizomycetes (mildews and yeasts), used as i starter cultures in order to obtain the blue-veining of the cheeses, which foresees a double safety level relating to the absence of gluten.

An aim of the present invention is to provide the use of a gluten-free spore or mildew culture belonging to the genus *Penicillium* in a process for the preparation of a gluten-free blue-veined dairy products, as claimed in claim 1.
Another aim of the present invention is to provide a method for the preparation of culture media capable of overcoming the limits of the known art and being able to be intended for persons affected by celiac disease in a safe way.

These and other aims, which will result apparent from the following detailed description, have been attained by the Applicant which has improved a methodology which, includes a double safety level relating to the absence of gluten in the culture media.

In particular, the Applicant has set up a methodology which foresees: a first process for the preparation of spore or mildew cultures developed in a selected naturally gluten-free culture substrate, capable of completely safeguarding the biological activity of the mildew spores and, therefore, the ability of ensuring an optimal blue-veining and, next, a second process which foresees an enzymatic treatment of said cultures. Mildews belonging to the species Penicillium *roqueforti*, preferably selected from the group deposited by the Applicant c/o the Collection centre DSMZ (Deutsche Sammlung von Mikroorganismen und Zellkulturen GmbH; Braunsweig - Germany) on 21.12.2005 form a subject of the present invention, in which said group consists of the microorganisms identified by said Centre, with the accession numbers reported in the following Table 1:

| | |
|---|---|
| Penicillium roqueforti | DSM 17815 |
| Penicillium roqueforti | DSM 17816 |
| Penicillium roqueforti | DSM 17817 |
| Penicillium roqueforti | DSM 17818 |
| Penicillium roqueforti | DSM 17819 |
| Penicillium roqueforti | DSM 17820 |
| Penicillium roqueforti | DSM 17821 |
| Penicillium roqueforti | DSM 17822 |
| Penicillium roqueforti | DSM 17823 |
| Penicillium roqueforti | DSM 17824 |
| Penicillium roqueforti | DSM 17825 |
| Penicillium roqueforti | DSM 17826 |
| Penicillium roqueforti | DSM 17827 |
| Penicillium roqueforti | DSM 17828 |
| Penicillium roqueforti | DSM 17829 |
| Penicillium roqueforti | DSM 17830 |

as reported in the appended independent claim.

A method for the preparation of a gluten-free spore or mildew culture, selected from the aforesaid group, a composition including at least one of said cultures and the use of at least one of said cultures for the preparation of a blue-veined dairy product intended for people with celiac disease form then an aim of the present invention, having the features as reported in the appended claims.

The blue-veining, that is the veins with a more or less intense green colour typical of the Gorgonzola (Italy), Roquefort, Bresse Blue, Blue d'Auvergne, Sassenage and other (France), Stilton (England), Danablue (Denmark), Cabrales (Spain) and Bluecheese (USA), is obtained by the growth of a mildew belonging to the genus *Penicillium* (species *Penicillium roqueforti*), which is voluntarily added to the milk at the time of the processing, together with milk enzymes and the rennet. The mildew strains, the spores amounts and the processing techniques vary from cheese to cheese, but all the typologies are characterized by a kind of lactic-rennet curdling, with a prevailing late lactic nature, with a wet and particularly soft and splitted curdling, with slow and progressive drainage. The enzymatic action of a mixed microflora (lactic bacteria, mildew and often yeasts) allows to obtain in relatively short times, very proteolyzed pastes (with a maturation coefficient of about 65%) characterized by the presence of various aromatic substances for the lipolytic action carried out by the Penicillium on the fat matter. The splittings, indispensable for the growth of the mildew, are a consequence both of the acidification carried out by the lactic enzymes and of the development of carbon dioxide produced by heterofermented yeasts or bacteria.

During the maturation the forms are subjected, in two different stages, to perforation of both the faces with proper needles. The operation has the purpose of allowing the air and therefore oxygen inlet within the splittings existing in the paste.

Only in an aerated environment, the *Penicillium* spores are able to develop themselves, by forming first an interlacement of hyphas (mycelium) which invade the holes and the splits and subsequently produce the conidiophores which carry the spores, with a more or less intens green/blue colour.

It is the colour of the spores which characterizes the typical aspect of the Gorgonzola and all the other blue-veined cheeses, while the proteolytic and the lipolytic actions of the mildew cause the maturation of the cheese and the formation of the peculiar sensory features.

The spore cultures of the different strains of *Penicillium roqueforti* used in the processing are prepared by specialized laboratories, in a liquid or freeze dried form, according to the technique known to the skilled in the art.

Indeed, the preparation of an intermediate culture is performed, which foresees a seeding of the spores of the strain *Penicillium* (from a tube of the Working Cell Bank) in a proper agar culture medium (for example agar potato, agar potato-dextrose, agar malt extract) and subsequent incubation at 22-28°C for 5-10 days, for the purpose of increasing the number thereof.

Once the growth and the spores formation of the intermediate culture (usually a week) in one of the culture media above described is ended, with a sterile pipette the spores are removed and collected from the surface of the agar medium.

In a particularly preferred embodiment, said intermediate culture is obtained by the growth of spores belonging to the species *Penicillium roqueforti*, selected from the group above described and claimed in the appended independent claim.

Said spores have been isolated from a natural habitat, preferably from forage essences.

The spores of the intermediate culture, after suspension in a proper sterile liquid medium (physiological solution or the culture broths themselves above described in the formulation without agar) form the inoculum for the production of the end culture. The Applicant has found to be useful to use, as a growth substrate, at least a naturally gluten-free substrate of vegetal origin.

In practice, the Applicant has found useful the use, as a growth substrate, of one or more substrates selected from the group including: naturally gluten-free rice, maize, potato, manioc, tapioca, chestnuts, peas, fava beans and legumes, in form of blown or exploded grains (such as pop-corns) , or in form of flours as such and/or precooked.

With reference to the tubers (potato, tapioca and manioca), they have to be previously cut in small cubes or strips, depending on the possibility; however, it is also possible to use their flours, both as such and precooked. On the contrary, the legumes (for example broad beans, peas, beans and chick peas) are used as such or in a dried form). However, it is possible to use any mixture of the raw materials listed so far. Advantageously, the substrate is selected from rice and/or maize; preferably in form of a blown or exploded substrate or in form of flour, used alone and/or in all the simple and/or complex foreseeable combinations. The preferred substrate, consisting of blown rice and/or exploded maize is introduced in pirex glass bottles with a large opening, which are subsequently closed with a bacteriological cotton and sterilized in autoclave at 121°C for 20 minutes.

Each bottle is then inoculated with a suspension of spores of the *Penicillium* culture and placed for the incubation, together with many other identical suspensions forming a single production lot, in thermostats with a controlled temperature varying from 20 to 28°C, as a function of the used strains.

The culture substrate nature and the incubation conditions allow the passage from the quiescent spore form to the vegetative form; the spore germinates and from each, cells (hyphas), which produce enzymes capable of transforming the complex substances existing in the above mentioned substrate in simpler molecules which are absorbed by the cells and determine a fast growth of the same, are formed.

Each hypha extends and segments itself bringing about two daughter cells which perpetrate the mechanism, giving rise in a few days to long filaments. From the more or less close interlacement of the hyphas, a kind of white-greyish-coloured felt is formed (mycelium), which then, when it reaches a well precise growth level and when the environmental conditions are advantageous, produces particular hyphas in form of a "brush" (conidiophores) with the fruit bodies which, in turn, carry the spores with a more or less intense green/blue colour.

The time required for the maintenance of the spores changes from strain to strain, but generally, using bread as a substrate, the cycle is ended after 12-21 days from the inoculum.

When the growth is completed, one or more substrates subject of the present invention, completely invaded and coated of mycelium and green spores, are removed from the bottle with the help of a sterile solution of water, salt and agar and subjected to crushing, homogenization and filtration in proper plants for the spores extraction. At the end of the process, a green/blue-coloured liquid is obtained, in which the spores of the produced strain *Penicillium* are suspended.

The extraction step is followed by a spores titre standardization step, an aim which is attained through addition of a further fraction of sterile solution in water, salt and agar until the same optical density of a reference sample of the strain, evaluated by nephelometric comparison, is reached. The method described so far already allows to certify the *Penicillium* culture thus manufactured as a "gluten-free" culture, having used in any step of the productive process no naturally gluten-containing ingredient, excipient or substance.

Nevertheless, in order to ensure a further safety level, the Applicant has advantageously associated with such production methodology a treatment with proteolytic enzymes capable of degrading the gluten, if any, coming from accidental cross-contaminations to be carried out on the spores suspension obtained from the substrates above described.

Therefore, with the present disclosure the Applicant is able to provide a methodology which foresees a double safety level, capable of also eliminating the possible risk due to the presence of gluten traces because of cross-contaminations occurred along the production chain of the raw materials used.

The enzymatic treatment, subject of the present disclosure, has been defined after having evaluated multiple proteolytic enzymes, both proteases and peptidases and their mixtures, such as trypsin, chymotrypsin, pan- creatin, pepsin, papain and bromelain.

Advantageously, pepsin and bromelain are used, or mixtures of the two enzymes in a ratio from 1:10 to 10:1 of the same; preferably, in a weight ratio from 1:5 to 5:1.

The pepsin is an enzyme contained in the gastric juice which promotes the digestion of the foods. The pepsin is a protease which works at best in an acid environment and is able to break the proteins in many different points and in disorder. Its hydrolytic action is due to an active site which contains two residuals of aspartic acid. It is extracted, in particular, from the gastric mucosa of the swines, or from the fourth stomach of the ruminants, in particular bovines. It is commercially available both in liquid and powdery form with a different titre, expressed in Enzymatic Units, or for the bovine type, used in the dairy industry in order to clot the milk and affect the maturation of the cheese, in MCU (Milk Clotting Units). The bromelain is a complex proteinase mixture generally extracted from the pineapple stem, even if similar enzymes are also present in its pulp. The bromelain is available in powder with an enzymatic activity expressed in GDU (Gelatin Digesting Units) or MCU (Milk Clotting Units). 1 MCU is equivalent to approximately 2/3 GDU.

The gluten hydrolysis takes place in a quite wide range of temperature, from 5 to 60°C; preferably, from 30 to 50°C. The pH value is between 1 to 7; preferably, from 4 to 5. In particular, the pH for the pepsin is between 1.8 to 6.0 and for the bromelain the pH is between 3.5 and 6.5.

Advantageously, the temperature is between 30 and 40°C; preferably, it is 37°C for the pepsin. Advantageously, the temperature is between 40 and 50°C; preferably, it is 45°C for the bromelain.

The hydrolysis time is between 5 to 60 hours; preferably from 15 to 50 hours.

For the definition of the working conditions with which the enzymatic treatment, suitable for eliminating every gluten presence in the mildew cultures produces with naturally gluten-free substances, has to be carry out, experimentations have been conducted, in which to such cultures known quantities of gluten have voluntarily been added, so as to bring the end concentration in a varying interval from 3 to 20 ppm. Such gluten concentrations stimulate the more serious situation that could occur following to cross-contaminations occurred in the production chain of the naturally gluten-free foods and substances, used as growth substrates of the mildew.

In the experimentations, carried out for the purpose of hydrolysing the voluntarily added gluten, liquid pepsin with a titre between 300 and 800 MCU, advantageously with a titre equal to 500 MC has been used, and powdered bromelain with a titre between 1500 to 3000 GDU, advantageously with a titre equal to 2,200 GDU.

Several tests have been carried out, varying from time to time the end concentration of the enzymes, used alone and in admixture, or the hydrolysis working conditions (temperature, pH of the mildew and action time) .

The complete removal of the maximum quantity of added gluten, equal to 20 ppm, has been preferably carried out with the simultaneous use of pepsin (with a titre equal to 500 MCU) and bromelain (with a titre equal to 2,200 MCU), for a total length of 20-28 hours, for example 24 hours, with a temperature between 24 and 30<0>C at a controlled pH between 4 and 5. After the enzymatic treatment and approval of the lot by the Quality Control, the packaging in sterile bottles is performed.

Based on the European rule which will come into force at the end of 2005, only a blue-veined cheese produced with mildews manufacture with the technology subject of the present invention will give absolute guarantees about the absence of gluten therein. For this cheese, therefore, any mention in the label concerning the presence of gluten will be unnecessary.

While, concerning the raw materials used as growth substrates, a sufficient number of data have already been reported, some preferred embodiments relating to the enzymatic treatment are listed below by mere way of example which does not limit the importance of the present invention.

In these preferred embodiments, the working conditions are such that the biological activity of the mildew spores, and therefore its ability of ensuring an optimal blue-veining and maturation of the blue cheeses are protected.

For example, in a preferred embodiment it is used: a) bromelain concentration: from 0.1 to 0.4 g/liter of mildew (equal to 220-880 GD/l) hydrolysis pH: 4.4-4.6; hydrolysis temperature: 22-28°C (for example 24°C); hydrolysis length: 24-48 hours.

For example, in another preferred embodiment it is used: b) pepsin concentration: from 0.2 to 0.4 g/liter of mildew (equal to 100-200 MCU/1): hydrolysis pH: 4.4-4.6; hydrolysis temperature: 22-28°C (for example 24°C); hydrolysis length: 24-48 hours.

For example, in still another preferred embodiment it is used: c) bromelain concentration: from 0.05 to 0.2 g/liter of mildew (equal to 110-440 GDU/1); pepsin concentration: from 0.1 to 0.2 ml/liter of mildew (equal to 50- 100 MC/1): hydrolysis pH: 4.4-4.6; hydrolysis temperature: 22-28°C (for example 24°C); hydrolysis length: 24-48 hours.

Advantageously, with the method subject of the present disclosure, the gluten end quantity determined with the more sensitive test currently commercially available, is always resulted non determinable. In this case, it is possible to ensure the absence of gluten in the cultures produced with the methodology of the present disclosure. The analytical controls relating to the gluten concentrations have been carried out with the ELISA RIDASCREEN^{®} Gliadin kit of the R-Biopharm A, Darmstadt, Germany, for the quantitative analysis of the gliadin and the corresponding prolamine. From the journal "Approaches to Establish Thresholds for Major Food Allergens and for Gluten in Fodd" - Draft Report of the U.S. Food and Drug Administration of June 2005, it can be evicted that the analytical kit used for determining the gluten concentration is one of the few confirmed, the most sensitive, quantitative and specific for the different gliadins.

### Further embodiments are as follows:

A preferred embodiment of the present disclosure refers to spore or mildew of the species *Penicillium roqueforti* selected from the group consisting of the microorganisms as disclosed above in Table 1.

Another preferred embodiment of the present disclosure refers to a method for the preparation of a composition comprising at least a gluten-free spore or mildews culture which is **characterized in that** said culture is of the genus *Penicillium* and in that it is obtained by a gluten-free culture substrate.

Another preferred embodiment of the present disclosure refers to said method, wherein the spore or mildews culture is of the species *Penicillium roqueforti*.

Another preferred embodiment of the present disclosure refers to said method, wherein said culture is selected from the group consisting of the microorganisms as disclosed above in Table 1.

Another preferred embodiment of the present disclosure refers to said method, wherein the culture substrate is selected from those of vegetal origin.

Another preferred embodiment of the present disclosure refers to said method, wherein the culture substrate is selected from the group including: naturally gluten-free rice, maize, potato, manioca, tapioca, chestnuts, peas, broad beans, beans and legumes or their mixtures.

Another preferred embodiment of the present disclosure refers to said method, wherein the culture substrate is a mixture including rice and maize.

Another preferred embodiment of the present disclosure refers to said method, wherein the substrate is in form of blown or exploded grains or in form of flours as such and/or precooked.

Another preferred embodiment of the present disclosure refers to said method that includes at least a step in which at least a substrate, as defined above, is inoculated with an intermediate spore suspension of the species *Penicillium roquefort*; preferably, said spores are selected from the group of the microorganisms as disclosed above in Table 1.

Another preferred embodiment of the present disclosure refers to said method, wherein the inoculation is carried out for the required time and under such temperature conditions that the maturation of the spores is allowed.

Another preferred embodiment of the present disclosure refers to said method, wherein a separation step of a liquid solution, containing the spore culture, from the used substrate is further foreseen.

Another preferred embodiment of the present disclosure refers to said method, wherein a dehydration step of said solution to obtain the spore culture in a freeze-dried, sprayed or powdery form is further foreseen.

A further preferred embodiment of the present disclosure refers to a composition comprising at least a gluten-free spore or mildew culture as disclosed above in Table 1; preferably, said spores are selected from the group of microorganisms as disclosed above.

Another preferred embodiment of the present disclosure refers to a process for the preparation of a blue-veined dairy product including at least a step in which said composition including at least a gluten-free spore culture is used.

A further preferred embodiment of the present disclosure refers to a blue-veined dairy product obtained according to the process disclosed above.

A preferred embodiment of the present invention refers to use of a gluten-free spore culture of the genus *Penicillium*, prepared according to the process disclosed above, for the preparation of a blue-veined dairy product intended for people affected by celiac disease; preferably, said spores belong to the species *Penicillium roqueforti*; more preferably, they are selected from the group according to Table 1.

A further preferred embodiment of the present invention refers to use of a culture substrate selected from the group including: naturally gluten-free rice, maize, potato, manioca, tapioca, chestnuts, peas, broad beans, beans and legumes or their mixtures for the preparation of a gluten-free spore or mildew culture of the genus *Penicillium*, preferably, of the species *Penicillium roqueforti*; more preferably, said spores are selected from the group according to Table 1.

A further preferred embodiment of the present disclosure refers to a method for the preparation of a composition including at least a gluten-free spore or mildew culture, said method being **characterized in that** said composition is prepared according to the method disclosed above and in that it is further subjected to an enzymatic treatment for removing gluten traces, if any, existing in this latter composition.

A further preferred embodiment of the present disclosure refers to said method, wherein said spores or mildews belong to the genus *Penicillium*; preferably to the species *Penicillium roqueforti*; more preferably, they are selected from the group according to Table 1.

A further preferred embodiment of the present disclosure refers to said method, wherein the possible traces of gluten existing in said composition to be treated derive from accidental, unintentional or cross-contaminations.

A further preferred embodiment of the present disclosure refers to said method, wherein the enzymatic treatment foresees the use of at least a proteolytic enzyme.

A further preferred embodiment of the present disclosure refers to said method, wherein the proteolytic enzyme is selected from the group including: proteases and peptidases.

A further preferred embodiment of the present disclosure refers to said method, wherein the proteases and peptidases are selected from the group including: trypsin, chymotrypsin, pancreatin, pepsin, papain and bromelain; preferably, the proteases and the peptidases are selected from pepsin and/or bromelain.

A further preferred embodiment of the present disclosure refers to said method, wherein said at least one proteolytic enzyme added to the composition, prepared according to the method disclosed above, hydrolyzes the gluten traces, if any, existing in this latter composition.

A further preferred embodiment of the present disclosure refers to said method, wherein hydrolysis takes place at a pH value between 1 and 7, preferably from 4 to 5; at a temperature between 5 and 60°C, preferably from 30 to 50°C and in a time between 5 and 60 hours, preferably from 15 to 50 hours.

A further preferred embodiment of the present disclosure refers to said method, wherein hydrolysis is carried out with pepsin and/or bromelain under the following conditions: pH between 4.4 and 4.6; temperature between 22 and 28; and a time between 24 and 48 hours.

A further embodiment of the present disclosure refers to a composition including at least a gluten-free spore or mildew culture obtained according to the method disclosed above; preferably, said spores or mildews are selected from the group according to Table 1.

A further preferred embodiment of the present disclosure refers to a method for the preparation of a blue-veined dairy product including at least a step in which a composition including at least a gluten-free spore or mildew culture as disclosed above is used.

A further embodiment of the present disclosure refers to a blue-veined dairy product obtained according to the process disclosed above.

A further embodiment of the present invention refers to use of a gluten-free spore culture of the genus *Penicillium* prepared according to said method, for the preparation of a blue-veined dairy product intended for people affected by celiac disease; preferably, said spores belong to the species *Penicillium roqueforti*; more preferably, they are selected from the group according to Table 1.

## Claims

1. Use of a gluten-free spore or mildew culture belonging to the genus *Penicillium* in a process for the preparation of a gluten-free blue-veined dairy product by cultivating at least a spore or mildew culture belonging to the genus *Penicillium* in a gluten-free culture substrate of vegetal origin selected from the group including rice, maize, potato, manioca, tapioca, chestnuts, peas, fava beans, and legumes or their mixtures.

2. The use according to claim 1, wherein said at least a spore or mildew culture belonging to species *Penicillium roqueforti.*

3. The use according to claim 2, wherein said at least a spore or mildew of the species *Penicillium roqueforti* is selected from the group consisting of the following microorganisms:
| | |
|---|---|
| ***Penicillium roqueforti*** | **DSM 17815** |
| ***Penicillium roqueforti*** | **DSM 17816** |
| ***Penicillium roqueforti*** | **DSM 17817** |
| ***Penicillium roqueforti*** | **DSM 17818** |
| ***Penicillium roqueforti*** | **DSM 17819** |
| ***Penicillium roqueforti*** | **DSM 17820** |
| ***Penicillium roqueforti*** | **DSM 17821** |
| ***Penicillium roqueforti*** | **DSM 17822** |
| ***Penicillium roqueforti*** | **DSM 17823** |
| ***Penicillium roqueforti*** | **DSM 17824** |
| ***Penicillium roqueforti*** | **DSM 17825** |
| ***Penicillium roqueforti*** | **DSM 17826** |
| ***Penicillium roqueforti*** | **DSM 17827** |
| ***Penicillium roqueforti*** | **DSM 17828** |
| ***Penicillium roqueforti*** | **DSM 17829** |
| ***Penicillium roqueforti*** | **DSM 17830** |
which were deposited by the Applicant c/o the Collection centre DSMZ (Deutsche Sammlung von Mikroorganismen und Zellkulturen GmbH; Braunsweig - Germany) on 21.12.2005.

4. The use according to claim 1, wherein said culture substrate is in form of blown or exploded grains or in form of flours as such and/or precooked.

5. The use according to claim 1, wherein said gluten-free culture substrate is selected from rice and/or maize.

6. The use according to claim 5, wherein said gluten-free culture consists of blown rice and/or exploded maize.

## Patentansprüche

1. Verwendung einer glutenfreien Sporen- oder Schimmelkultur der Gattung Penicillium in einem Verfahren zur Herstellung eines glutenfreien Blauschimmel-Milchprodukts durch Kultivieren wenigstens einer Sporen- oder Schimmelkultur der Gattung Penicillium in einem glutenfreien Kultursubstrat pflanzlichen Ursprungs ausgewählt aus der Gruppe bestehend aus Reis, Mais, Kartoffeln, Maniok, Tapioka, Esskastanien, Erbsen, dicken Bohnen und Hülsenfrüchten oder deren Mischungen.

2. Verwendung gemäß Anspruch 1, worin die wenigstens eine Sporen- oder Schimmelkultur zur Spezies Penicillium roqueforti.

3. Verwendung gemäß Anspruch 2, worin die wenigstens eine Sporen- oder Schimmelkultur der Spezies Penicillium roqueforti ausgewählt ist aus der Gruppe bestehend aus den folgenden Mikroorganismen
| | |
|---|---|
| Penicillium roqueforti | DSM 17815 |
| Penicillium roqueforti | DSM 17816 |
| Penicillium roqueforti | DSM 17817 |
| Penicillium roqueforti | DSM 17818 |
| Penicillium roqueforti | DSM 17819 |
| Penicillium roqueforti | DSM 17820 |
| Penicillium roqueforti | DSM 17821 |
| Penicillium roqueforti | DSM 17822 |
| Penicillium roqueforti | DSM 17823 |
| Penicillium roqueforti | DSM 17824 |
| Penicillium roqueforti | DSM 17825 |
| Penicillium roqueforti | DSM 17826 |
| Penicillium roqueforti | DSM 17827 |
| Penicillium roqueforti | DSM 17828 |
| Penicillium roqueforti | DSM 17829 |
| Penicillium roqueforti | DSM 17830 |
die vom Anmelder am 21.12.2005 c/o Sammelzentrum DSMZ (Deutsche Sammlung von Mikroorganismen und Zellkulturen GmbH; Braunschweig - Deutschland) hinterlegt worden sind.

4. Verwendung gemäß Anspruch 1, wobei das Kultursubstrat in Form von aufgeplatzten oder aufgesprengten Getreidekörnern oder in Form von Mehl als solchem und/oder vorgekocht vorliegt.

5. Verwendung gemäß Anspruch 1, worin das glutenfreie Kultursubstrat ausgewählt ist aus Reis und/oder Mais.

6. Verwendung gemäß Anspruch 5, wobei die glutenfreie Kultur aus aufgeplatztem Reis und/oder aufgesprengtem Mais besteht.

## Revendications

1. Utilisation d'une culture de spores ou mildious sans gluten appartenant au genre *Penicillium* dans un procédé pour la préparation d'un produit laitier persillé sans gluten par culture d'au moins une culture de spores ou mildious appartenant au genre *Penicillium* dans un substrat de culture sans gluten d'origine végétale choisi parmi le groupe incluant le riz, le maïs, la pomme de terre, le manioc, le tapioca, les châtaignes, les pois, les fèves, et les légumineuses ou leurs mélanges.

2. Utilisation selon la revendication 1, dans laquelle ladite au moins une culture de spores ou mildious appartient à l'espèce *Penicillium roqueforti.*

3. Utilisation selon la revendication 2, dans laquelle ladite/ledit au moins une/un spore ou mildiou de l'espèce *Penicillium roqueforti* est choisi(e) parmi le groupe consistant en les microorganismes suivants :
| | |
|---|---|
| *Penicillium roqueforti* | DSM 17815 |
| *Penicillium roqueforti* | DSM 17816 |
| *Penicillium roqueforti* | DSM 17817 |
| *Penicillium roqueforti* | DSM 17818 |
| *Penicillium roqueforti* | DSM 17819 |
| *Penicillium roqueforti* | DSM 17820 |
| *Penicillium roqueforti* | DSM 17821 |
| *Penicillium roqueforti* | DSM 17822 |
| *Penicillium roqueforti* | DSM 17823 |
| *Penicillium roqueforti* | DSM 17824 |
| *Penicillium roqueforti* | DSM 17825 |
| *Penicillium roqueforti* | DSM 17826 |
| *Penicillium roqueforti* | DSM 17827 |
| *Penicillium roqueforti* | DSM 17828 |
| *Penicillium roqueforti* | DSM 17829 |
| *Penicillium roqueforti* | DSM 17830 |
qui ont été déposés par le Demandeur aux bons soins du Centre de Collecte DSMZ (Deutsche Sammlung von Mikroorganismen und Zellkulturen GmbH ; Braunsweig Allemagne) le 21/12/2005.

4. Utilisation selon la revendication 1, dans laquelle ledit substrat de culture est sous la forme de grains soufflés ou éclatés ou sous la forme de farines en l'état et/ou précuites.

5. Utilisation selon la revendication 1, dans laquelle ledit substrat de culture sans gluten est choisi parmi le riz et/ou le maïs.

6. Utilisation selon la revendication 5, dans laquelle ladite culture sans gluten consiste en du riz soufflé et/ou du maïs éclaté.
